# EUROPEAN PATENT APPLICATION

(11) **EP 3 017 813 A1**
(43) Date of publication of application: **11.05.2016**
(21) Application number: 14191735.1
(22) Date of filing: 04.11.2014
(51) Int. Cl.: A61K 31/192, A61K 31/41, A61K 31/559, C12N 5/079, C12N 5/0793, A61P 25/00, A61P 17/04

(54) **BLT2 agonists for the treatment of pain**

(71) Applicant: Fraunhofer Gesellschaft zur Förderung der angewandten Forschung e.V., 80686 München (DE); Johann Wolfgang Goethe-Universität, Frankfurt am Main, 60323 Frankfurt am Main (DE)
(72) Inventor: Zinn, Sebastian, 60528 Frankfurt (DE); Scholich, Klaus, 61449 Steinbach (DE); Geisslinger, Gerd, 65812 Bad Soden (DE); de Bruin, Natasja, 60488 Frankfurt am Main (DE)
(74) Representative: Krauss, Jan

(57) **Abstract**

The present invention pertains to novel analgesics useful for treating pain. BLT2 agonists were found to desensitize TRPV1 mediated signalling in sensory neurons. Thus the invention provides BLT2 agonists as novel pain therapeutics. Additional aspects of the invention pertain to combinations of BLT2 agonists with BLT1 antagonists for treating pain in subjects. Pharmaceutical compositions and kits comprising the new analgesics of the invention are furthermore provided.

## Description

### FIELD OF THE INVENTION

The present invention pertains to novel analgesics useful for treating pain. BLT2 agonists were found to desensitize TRPV mediated signalling in sensory neurons. Thus the invention provides BLT2 agonists as novel pain therapeutics. Additional aspects of the invention pertain to combinations of BLT2 agonists with BLT1 antagonists for treating pain in subjects. Pharmaceutical compositions and kits comprising the new analgesics of the invention are furthermore provided.

### DESCRIPTION

Pain is a complex subjective sensation reflecting real or potential tissue damage and the affective response to it. Acute pain is a physiological signal indicating a potential or actual injury. Chronic pain can either be somatogenetic (organic) or psychogenic. Chronic pain is frequently accompanied or followed by vegetative signs, which often result in depression. Chronic pain results in individual suffering and social economic costs of tremendous extent. Existing pharmacological pain therapies are widely unsatisfying both in terms of efficacy and of safety. Somatogenetic pain may be of nociceptive origin, inflammatory or neuropathic. Nociceptive pain is judged to be commensurate with ongoing activation of somatic or visceral pain-sensitive nerve fibers. Neuropathic pain results from dysfunction in the nervous system that is sustained by aberrant somatosensory processes in the peripheral nervous system.

Neuropathic pain is a persistent or chronic pain syndrome that can result from damage to the nervous system, the peripheral nerves, the dorsal root ganglion, dorsal root, or to the central nervous system. Neuropathic pain syndromes include allodynia, various neuralgias such as post herpetic neuralgia and trigeminal neuralgia, phantom pain, and complex regional pain syndromes, such as reflex sympathetic dystrophy and causalgia. Causalgia is often characterized by spontaneous burning pain combined with hyperalgesia and allodynia. Tragically there is no existing method for adequately, predictably and specifically treating established neuropathic pain as present treatment methods for neuropathic pain consists of merely trying to help the patient cope through psychological or occupational therapy, rather than by reducing or eliminating the pain experienced. Treatment of neuropathic or chronic pain is a challenge for physicians and patients since there are no medications that specifically target the condition, and since the medications presently used result in only little relief and are based on their efficacy in acute pain conditions or on their efficacy on relieving secondary effects like anxiety and depression. Incidence of chronic pain is increasing in society and its burden on society is huge in both health care and lost productivity. Currently there are no scientifically validated therapies for relieving chronic pain. As a result, the health community targets 'pain management' where multi-modal therapies are used concurrently with the hope of providing some improvement in quality of life. Thus, there is an urgent need for drugs that can relieve chronic pain.

Recent studies identified members of the transient receptor potential-family of ion channels (TRPV1, TRPA1 and TRPV4) as contributors to both mechanical and cold allodynia during oxaliplatin and paclitaxel-induced neuropathy. Activation or sensitization of TRPV1 and TRPA1 can lead to enhanced release of CGRP and substance P both of which can cause neurogenic inflammation and recruitment of T-cells.

Capsaicin is a highly selective agonist for transient receptor potential vanilloid 1 receptor (TRPV1; formerly known as vanilloid receptor 1 (VR1)), a ligand-gated, non-selective cation channel preferentially expressed on small-diameter sensory neurons, especially those C-fibers which specialize in the detection of painful or noxious sensations. TRPV1 responds to noxious stimuli including capsaicin, heat, and extracellular acidification, and will integrate simultaneous exposures to these stimuli. The initial effect of the activation of TRPV1-expressing (capsaicin-sensitive) nociceptors are burning sensations, hyperalgesia, allodynia, and erythema. However, after prolonged exposure to low-concentration capsaicin or single exposures to high-concentration capsaicin or other TRPV1 agonist, the small-diameter sensory axons become less sensitive to a variety of stimuli, including capsaicin or thermal stimuli. This prolonged exposure is also characterized by reduced pain responses. These later-stage effects of capsaicin are frequently referred to as "desensitization" and are the rationale for the development of local capsaicin formulations for the treatment of various pain syndromes and other conditions.

Up to now, two classes of analgesics are mainly employed for the treatment of pain: Non-opioid analgesics, mostly acetaminophen and NSAIDS (non-steroidal anti-inflammatory drugs) and opioid (narcotic) agonists (wherein "opioid" is a generic term for natural or synthetic substances that bind to specific opioid receptors in the CNS, producing an agonist action). Unfortunately both analgesic classes, opioids and non-opioids, have several unwanted side effects. The most serious side effects of opioids are the possibility of inhibition of the respiratory system and after long-term treatment the possibility of addiction. NSAIDs, a major class of non-opioids, on the other hand, can induce a variety of gastrointestinal complications such as ulcers and bleeding, but also kidney damage (Scholich and Geisslinger, 2006).

Hence, until this day there is no specific therapy for neuropathic pain available. The present invention seeks to provide novel analgesics that overcome the problems associated with opioids and NSAIDs.

The above problem is solved in a first aspect by a Leukotriene B4 receptor 2 (BLT2) agonist for use in the inhibition of a neurological sensation in a subject, wherein the neurological sensation is mediated by the activation of transient receptor potential cation channel subfamily V member 1 (TRPV1).

Leucotriene (LT) B4 is known to induce pain and to be a powerful chemotactic agent for neutrophil granulocytes thereby promoting inflammation and inducing pain. Two G-protein coupled receptors, BLT1 and BLT2, have been shown to be activated by LTB4. It was shown previously that BLT1 mediates the chemotactic and pain stimulating effects of LTB4.

Here it was surprisingly discovered that the use of a BLT2 agonist reduces TRPV1 sensitization - a major mediator of nociception - induced by various stimuli such as LTB4, PGE2 receptor (EP4) ligand and bradykinin induced protein kinase A (PKA) activation. The inventors have developed a novel tool to reduce TRPV1 mediated activation of sensory neurons and therefore provided new compounds that are useful as analgesics.

As used herein, the term "BLT2 agonist" means a substance that affects an increase in the amount or rate of BLT2 expression or activity. Such a substance can act directly, for example, by binding to BLT2 and increasing the amount or rate of BLT2 expression or activity. A BLT2 agonist can also increase the amount or rate of BLT2 expression or activity, for example, by binding to BLT2 in such a way as to enhance or promote interaction of BLT2 with a BLT2 ligand; BLT2 activation may be affected by binding to BLT2 and modifying it, such as inducing a conformational change, or removal or addition of a moiety; and by binding to BLT2 and enhancing its stability. A BLT2 agonist can also act indirectly, for example, by binding to a regulatory molecule or gene region so as to modulate regulatory protein or gene region function and affect an increase in the amount or rate of a BLT2 expression or activity. Thus, a BLT2 agonist can act by any mechanisms that result in an increase in the amount or rate of BLT2 expression or activity.

A BLT2 agonist can be, for example, a naturally or non-naturally occurring macromolecule, such as a polypeptide, peptide, peptidomimetic, nucleic acid, carbohydrate or lipid. A BLT2 agonist further can be an antibody, or antigen-binding fragment thereof, such as a monoclonal antibody, humanized antibody, chimeric antibody, minibody, bifunctional antibody, single chain antibody (scFv), variable region fragment (Fv or Fd), Fab or F(ab)2. A BLT2 agonist can also be a polyclonal antibody specific for BLT2. A BLT2 agonist further can be a partially or completely synthetic derivative, analog or mimetic of a naturally occurring macromolecule, or a small organic or inorganic molecule.

A BLT2 agonist that is an antibody can be, for example, an antibody that binds to BLT2 and activates receptor signalling by mimicking ligand binding, or alters the activity of a molecule that regulates BLT2 expression or activity, such that the amount or rate of BLT2 expression or activity is increased. An antibody useful in a method of the invention can be a naturally occurring antibody, including monoclonal or polyclonal antibodies or fragments thereof, or a non-naturally occurring antibody, including but not limited to a single chain antibody, chimeric antibody, bifunctional antibody, complementarity determining region-grafted (CDR-grafted) antibody and humanized antibody or an antigen-binding fragment thereof.

BLT2 agonists in accordance with the present invention are also expression constructs expressing BLT2 proteins or functional fragments thereof. The term "expression construct" means any double-stranded DNA or double-stranded RNA designed to transcribe an RNA, e.g., a construct that contains at least one promoter operably linked to a downstream gene or coding region of interest (e.g., a cDNA or genomic DNA fragment that encodes a protein, or any RNA of interest) - in particular a BLT2 gene or a fragment thereof. Transfection or transformation of the expression construct into a recipient cell allows the cell to express RNA or protein encoded by the expression construct. An expression construct may be a genetically engineered plasmid, virus, or an artificial chromosome derived from, for example, a bacteriophage, adenovirus, retrovirus, poxvirus, or herpesvirus, or further embodiments described under "expression vector" below. An expression construct can be replicated in a living cell, or it can be made synthetically. For purposes of this application, the terms "expression construct", "expression vector", "vector", and "plasmid" are used interchangeably to demonstrate the application of the invention in a general, illustrative sense, and are not intended to limit the invention to a particular type of expression construct. Further, the term expression construct or vector is intended to also include instances wherein the cell utilized for the assay already endogenously comprises such DNA sequence. In particular preferred are expression constructs that allow for a BLT2 expression specifically in sensory neurons.

As used herein, "expression" includes the process by which polynucleotides are transcribed into mRNA and translated into peptides, polypeptides, or proteins. If the polynucleotide is derived from genomic DNA, expression may include splicing of the mRNA, if an appropriate eukaryotic host is selected. Regulatory elements required for expression include promoter sequences to bind RNA polymerase and transcription initiation sequences for ribosome binding. For example, a bacterial expression vector includes a promoter such as the lac promoter and for transcription initiation the Shine-Dalgarno sequence and the start codon AUG (Sambrook, J., Fritsh, E. F., and Maniatis, T., Molecular Cloning: A Laboratory Manual. 2nd, ed., Cold Spring Harbor Laboratory, Cold Spring Harbor Laboratory Press, Cold Spring Harbor, N.Y., 1989). Similarly, a eukaryotic expression vector includes a heterologous or homologous promoter for RNA polymerase II, a downstream polyadenylation signal, the start codon AUG, and a termination codon for detachment of the ribosome. Such vectors can be obtained commercially or assembled by the sequences described in methods well known in the art, for example, the methods described below for constructing vectors in general.

In some embodiments a BLT2 agonist is a compound that when brought into contact with a peripheral neuron is capable of decreasing TRPV1 sensitization via LTB4 or PKA. For example this can be tested in an experimental setup as provided in the Examples 3 and 4 as described herein below. In brief, DRG neurons are incubated with a candidate BLT2 agonist together with LTB4 or an activator of PKA, such as ONOae329. Upon stimulation with capsaicin the intracellular calcium increase is measured. If the incubation with the candidate BLT2 agonist reduces intracellular calcium increase compared to a control compound, then the candidate compound is a BLT2 agonist in accordance with the present invention.

In preferred embodiments of the invention a BLT2 agonist is not a BLT1 agonist, preferably the BLT2 agonist is a selective BLT2 agonist, or even more preferably a BLT2 agonist that is also a BLT1 antagonist.

A preferred BLT2 agonist of the invention is 4'-[[(1-oxopentyl)phenylamino]methyl]-[1,1'-biphenyl]-2-carboxylic acid (CAY10583), as well as derivatives, analogs and salts of this compound.

A BLT2 agonist may be selected from any BLT2 agonist as disclosed in WO 2005/102388.

In preferred embodiments of the invention the neurological sensation mediated by TRPV1 activation is pain of any sorts.

The term "pain" as used herein refers to an unpleasant sensation. For example, the subject experiences discomfort, distress or suffering. It is known to one skilled in the art that various painful conditions may be classified according to broadly opposing or otherwise useful categories. Examples of opposing categories include; nociceptive pain versus non-nociceptive pain, and acute pain versus chronic pain. Examples of other common categories of pain used by those skilled in the art include neuropathic pain and phantom pain.

The term "nociception" as used herein refers to the transduction of noxious or potentially injurious stimuli into a sensation.

The term "nociceptive pain" as used herein refers to pain caused by activity in primary sensory pain fibers in the peripheral nervous system. Neurons in the peripheral nervous system that typically respond to noxious or painful stimuli are commonly referred to as nociceptors or nociceptive neurons. Yet further, the nociceptive pathways extend to the somatosensory cortex.

The term "non-nociceptive pain" as used herein refers to pain caused by activity in neurons in the central nervous system. Examples of neurons in the central nervous system that may cause non-nociceptive pain include neurons in the dorsal horn of the spinal cord such as interneurons and projection neurons, or neurons in parts of the brain known to be involved in pain sensation such as the rostral ventromedial medulla (RVM) and the periaqueductal grey (PAG).

The term "acute pain" as used herein refers to pain that is transient in nature or lasting less than 1 month. Acute pain is typically associated with an immediate injurious process such as soft tissue damage, infection, or inflammation, and serves the purpose of notifying the animal of the injurious condition, thus allowing for treatment and prevention of further injury.

The term "chronic pain" as used herein refers to pain that lasts longer than 1 month or beyond the resolution of an acute tissue injury or is recurring or is associated with tissue injury and/or chronic diseases that are expected to continue or progress. Examples of chronic diseases that are expected to continue or progress may include cancer, arthritis, inflammatory disease, chronic wounds, cardiovascular accidents, spinal cord disorders, central nervous system disorder or recovery from surgery.

The term "neuropathy" as used herein refers to any condition that adversely affects the normal functioning of the nervous system. Neuropathies can originate anywhere in the central or peripheral nervous system, but only in some cases does this produce neuropathic pain.

The term "neuropathic pain" as used herein refers to pain that result from damage to or abnormal function of the nervous system itself. It may exist independently of any form of tissue injury outside of the nervous system. Examples of conditions that may lead to neuropathic pain include disease (e.g., HIV, Herpes, Diabetes, Cancer, autoimmune disorders), acute trauma (surgery, injury, electric shock), and chronic trauma (repetitive motion disorders, chemical toxicity such as alcohol, chemotherapy, or heavy metals).

The term "phantom pain" as used herein refers to a condition whereby the patient senses pain in a part of the body that is either no longer physically present due to amputation, or is known to be completely insensate due to total peripheral nerve destruction.

The term "hyperalgesia" as used herein refers to an increased sensitivity to nociceptive or painful stimuli. The term "allodynia" as used herein describes a condition whereby normally non-noxious stimuli are perceived as painful. Both hyperalgesia and allodynia can be divided into primary and secondary categories or conditions. Primary hyperalgesia/allodynia is an increase in sensitivity to painful and previously non-painful stimuli in a region of the body that has undergone tissue injury. Secondary hyperalgesia/allodynia is an increase in pain sensitivity globally and requires descending input into the periphery from various pain processing centers in the brain.

Another embodiment of the invention further pertains to the use of a BLT2 agonist as described above, wherein said BLT2 agonist is used in combination with at least one additional compound inhibiting TRPV1-sensitization.

For the prevention or treatment of pain in accordance with the present invention, the BLT2 agonist is administered to the subject suspected to suffer from pain or suffering from pain. Furthermore preferred is the further administration of at least one additional compound inhibiting TRPV1-sensitization to the subject suspected to suffer from pain or suffering from pain.

In the context of the herein disclosed invention the at least one additional compound inhibiting TRPV1-sensitization is preferably a Leukotriene B4 receptor 1 (BLT1) antagonist or inhibitor.

The problem of the prior art pain treatments is furthermore solved by a combination for use in the prevention or treatment of pain, wherein the combination comprises (i) a BLT2 agonist as defined herein above and (ii) at least one additional compound inhibiting TRPV1-sensitization. Preferably the at least one additional compound inhibiting TRPV1-sensitization is a BLT1 antagonist or inhibitor (see above). Most preferably the combination comprises a BLT2 agonist and a BLT 1 antagonist.

The term "combination" means in this context an active substance combination of two or more active substances in a formulation and also as a combination in the sense of individual formulations of the active substances administered at specified intervals from one another in a therapeutic treatment. Thus the term "combination" shall include the clinical reality of a co-administration of two or more therapeutically effective compounds, as it is described in context of the present invention.

Co-administration: In the context of the present application, co-administration of two or more compounds is defined as administration of the two or more compounds to the patient within one year, including separate administration of two or more medicaments each containing one of the compounds as well as simultaneous administration whether or not the two or more compounds are combined in one formulation or whether they are in two or more separate formulations.

The combination of the invention in one embodiment includes that (i) and (ii) are combined by sequential or concomitant administration to a subject during said prevention or treatment, preferably wherein the antagonists and chemotherapeutics are concomitantly administered during said prevention or treatment.

As used herein, the term "BLT1-antagonist" means a substance that affects a decrease in the amount or rate of BLT1 expression or activity. Such a substance can act directly, for example, by binding to BLT1 and decreasing the amount or rate of BLT1 expression or activity. A BLT1-antagonist can also decrease the amount or rate of BLT1 expression or activity, for example, by binding to BLT1 in such a way as to reduce or prevent interaction of BLT1 with a BLT1 ligand; by binding to BLT1 and modifying it, such as by removal or addition of a moiety; and by binding to BLT1 and reducing its stability. A BLT1-antagonist can also act indirectly, for example, by binding to a regulatory molecule or gene region so as to modulate regulatory protein or gene region function and affect a de-crease in the amount or rate of BLT1 expression or activity. Thus, a BLT1-antagonist can act by any mechanisms that result in decrease in the amount or rate of BLT1 expression or activity.

A BLT1-antagonist can be, for example, a naturally or non-naturally occurring macromolecule, such as a polypeptide, peptide, peptidomimetic, nucleic acid, carbohydrate or lipid. A BLT1-antagonist further can be an antibody, or antigen-binding fragment thereof, such as a monoclonal antibody, humanized antibody, chimeric antibody, minibody, bifunctional antibody, single chain antibody (scFv), variable region fragment (Fv or Fd), Fab or F(ab)2. A BLT1-antagonist can also be polyclonal antibodies specific for BLT1. A BLT1-antagonist further can be a partially or completely synthetic derivative, analog or mimetic of a naturally occurring macromolecule, or a small organic or inorganic molecule.

A BLT1-antagonist that is an antibody can be, for example, an antibody that binds to BLT1 1 and inhibits binding to a BLT1 ligand, or alters the activity of a molecule that regulates BLT1 1 expression or activity, such that the amount or rate of BLT1 expression or activity is decreased. An antibody useful in a method of the invention can be a naturally occurring antibody, including monoclonal or polyclonal antibodies or fragment thereof, or a non-naturally occurring antibody, including but not limited to a single chain antibody, chimeric antibody, bifunctional antibody, complementarity determining region-grafted (CDR-grafted) antibody and humanized antibody or an antigen-binding fragment thereof.

A BLT1-antagonist that is a nucleic acid can be, for example, an anti-sense nucleotide sequence, an RNA molecule, or an aptamer sequence. An anti-sense nucleotide sequence can bind to a nucleotide sequence within a cell and modulate the level of expression of BLT1, BLT1 ligand or modulate expression of another gene that controls the expression or activity of BLT1. Similarly, an RNA molecule, such as a catalytic ribozyme, can bind to and alter the expression of the BLT1 gene, or other gene that controls the expression or activity of BLT1. An aptamer is a nucleic acid sequence that has a three dimensional structure capable of binding to a molecular target.

A BLT1-antagonist that is a nucleic acid also can be a double-stranded RNA molecule for use in RNA interference methods. RNA interference (RNAi) is a process of sequence-specific gene silencing by post-transcriptional RNA degradation, which is initiated by double-stranded RNA (dsRNA) homologous in sequence to the silenced gene. A suitable double-stranded RNA (dsRNA) for RNAi contains sense and antisense strands of about 21 contiguous nucleotides corresponding to the gene to be targeted that form 19 RNA base pairs, leaving overhangs of two nucleotides at each 3' end (Elbashir et al., Nature 411:494-498 (2001); Bass, Nature 411:428-429 (2001); Zamore, Nat. Struct. Biol. 8:746-750 (2001)). dsRNAs of about 25-30 nucleotides have also been used successfully for RNAi (Karabinos et al., Proc. Natl. Acad. Sci. USA 98:7863-7868 (2001). dsRNA can be synthesized in vitro and introduced into a cell by methods known in the art.

A preferred BLT1 antagonist according to the invention is 6-(6-(3R-hydroxy-1E,5Z-undecadien-1-yl)-2-pyridinyl)-1,5S-hexanediol (U75302). Other BLT1 and/or LTB4 antagonists (or inhibitors) useful in context of the present invention are the compounds LY293111, ONO4057, CP195543, CGS25019C, Biomed 101, BIIL284BS, DW1350, LY255283, and analogs of any of the foregoing.

In accordance with the invention alternative embodiments also pertain to a TRPV1 mediated sensation which is a taste sensation, itching of the skin, a burning sensation, or a nociceptive (pain) sensation. As already mentioned above, the pain can be selected from inflammatory pain, inflammatory hyperalgesia, hyperalgesia, neuropathic pain, migraine, cancer pain, visceral pain, osteoarthritis pain, chronic pain and post-surgical pain. However, neuropathic pain is most preferred.

Neuropathic pain may be selected from peripheral neuropathic pain syndrome, chemotherapy-induced neuropathy, complex regional pain syndrome, HIV sensory neuropathy, neuropathy secondary to tumor infiltration, painful diabetic neuropathy, phantom limb pain, postherpetic neuralgia, postmastectomy pain, trigeminal neuralgia, central neuropathic pain syndrome, central poststroke pain, multiple sclerosis pain, Parkinson disease pain, or spinal cord injury pain.

The invention provides in one further aspect a pharmaceutical composition for use in the prevention or treatment of pain, comprising a BLT2 agonist as defined herein, or a combination of compounds as defined herein above. The pharmaceutical compositions may further comprise a pharmaceutically acceptable carrier and/or excipient.

Pharmaceutical compositions and kits for treating or pre-venting pain form part of the present invention. In one embodiment, the composition comprises a BLT2 agonist as described above.

As used herein the language "pharmaceutically acceptable carrier" is intended to include any and all solvents, solubilizers, fillers, stabilizers, binders, absorbents, bases, buffering agents, lubricants, controlled release vehicles, diluents, emulsifying agents, humectants, lubricants, dispersion media, coatings, antibacterial or antifungal agents, isotonic and absorption delaying agents, and the like, compatible with pharmaceutical administration. The use of such media and agents for pharmaceutically active substances is well-known in the art. Except insofar as any conventional media or agent is incompatible with the active compound, use thereof in the compositions is contemplated. Supplementary agents can also be incorporated into the compositions. In certain embodiments, the pharmaceutically acceptable carrier comprises serum albumin.

The pharmaceutical composition of the invention is formulated to be compatible with its intended route of administration. Examples of routes of administration include parenteral, e.g., intrathecal, intra-arterial, intravenous, intradermal, subcutaneous, oral, transdermal (topical) and transmucosal administration.

Solutions or suspensions used for parenteral, intradermal, or subcutaneous application can include the following components: a sterile diluent such as water for injection, saline solution, fixed oils, polyethylene glycols, glycerine; propylene glycol or other synthetic solvents; antibacterial agents such as benzyl alcohol or methyl parabens; antioxidants such as ascorbic acid or sodium bisulfate; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, the injectable composition should be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the requited particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, and sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (e.g., a neuregulin) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Stertes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the pharmaceutical compositions are formulated into ointments, salves, gels, or creams as generally known in the art.

In certain embodiments, the pharmaceutical composition is formulated for sustained or controlled release of the active ingredient. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from e.g. Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art.

It is especially advantageous to formulate oral or parenteral compositions in dosage unit form for ease of administration and uniformity of dosage. Dosage unit form as used herein includes physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. The pharmaceutical compositions can be included in a container, pack, or dispenser together with instructions for administration.

The above problem of the invention is furthermore solved by a method for desensitization of TRPV1 in a cell, the method comprising the step of activation of BLT2. The activation of BLT2 in the cell is preferably affected by contacting the cell with an effective amount of a BLT2 agonist.

In this context a "cell" is preferably a BLT2 expressing cell, preferably wherein the cell is a neuron, preferably a peripheral neuron, more preferably a neuron of the dorsal root ganglion (DRG).

Some embodiments relate to the above method which is an *ex vivo* or *in vitro* method.

Another aspect of the herein described invention then relates to a method for desensitization of TRPV1 in a subject in need of such a treatment, the method comprising a step of administration of an effective amount of a BLT2 agonist to said subject. The desensitization is a desensitization of a TRPV1 mediated sensation, which is preferably selected from itching, burning, taste or pain.

In some embodiments of this aspect the method is for use in the treatment or prevention of pain in a subject.

Preferably the method comprises further administering to said subject a therapeutically effective amount of a TRPV1 antagonist and/or a BLT1 antagonist.

In context of the present invention the term "subject" preferably refers to a mammal, preferably a human. The subject of the invention may be at danger of suffering from pain, or suffer from pain, wherein the pain is as defined herein above.

Antagonists of the herein described invention are preferably selected from the group of compounds consisting of inhibitory RNA, inhibitory antibodies or fragments thereof, and/or small molecules.

In context of the invention it is also preferred that at least one additional therapeutic effective against pain, for example a morphine, an opioid or a non-opioid analgesic or other analgesic, is administered to said subject.

In another aspect of the invention there is provided a method for the prevention or treatment of pain in a subject, the method comprising the step of administering to said subject a therapeutically effective amount of a BLT2 agonist in accordance with the present invention.

The diseases treatable in context of the afore-described methods are described herein above.

During the treatment or prevention it is preferred that at least one additional therapeutic effective against pain is administered to said patient, such as other analgesics, for example an opioid or a non-opioid analgesic.

An additional aspect of the invention then relates to a method for decreasing sensitivity of Transient Receptor Potential Vanilloid 1 (TRPV1) in a subject, comprising administering to said subject a therapeutically effective amount of a BLT2 agonist.

The present invention will now be further described in the following examples with reference to the accompanying figures and sequences, nevertheless, without being limited thereto. For the purposes of the present invention, all references as cited herein are incorporated by reference in their entireties. In the Figures:

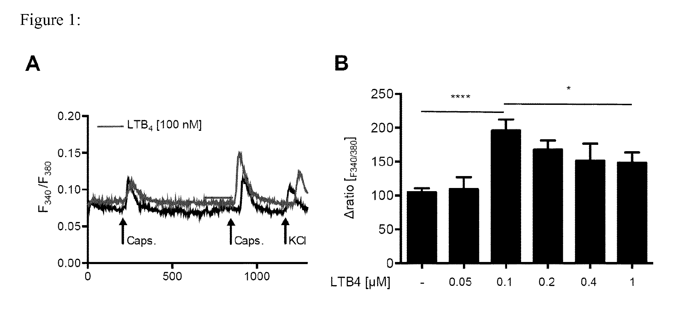

- **Figure 1:**: **LTB4 dose dependently sensitizes TRPV1 in DRG neurons.** (A) DRG neurons were double stimulated with capsaicin (200 nM, 15 s each) and incubated with either vehicle or LTB4 (100 nM) for two minutes prior to the second capsaicin stimulation. (B) Dose-dependent difference in ratio between the first and the second capsaicin response using the same protocol as described in (A). Data represent the mean ± SEM of the following number of neurons (n=20-86); ANOVA Kruskal-Wallis test; Dunn's multiple comparisons test *p<0.05, ****p<0.0001 student's t-test.

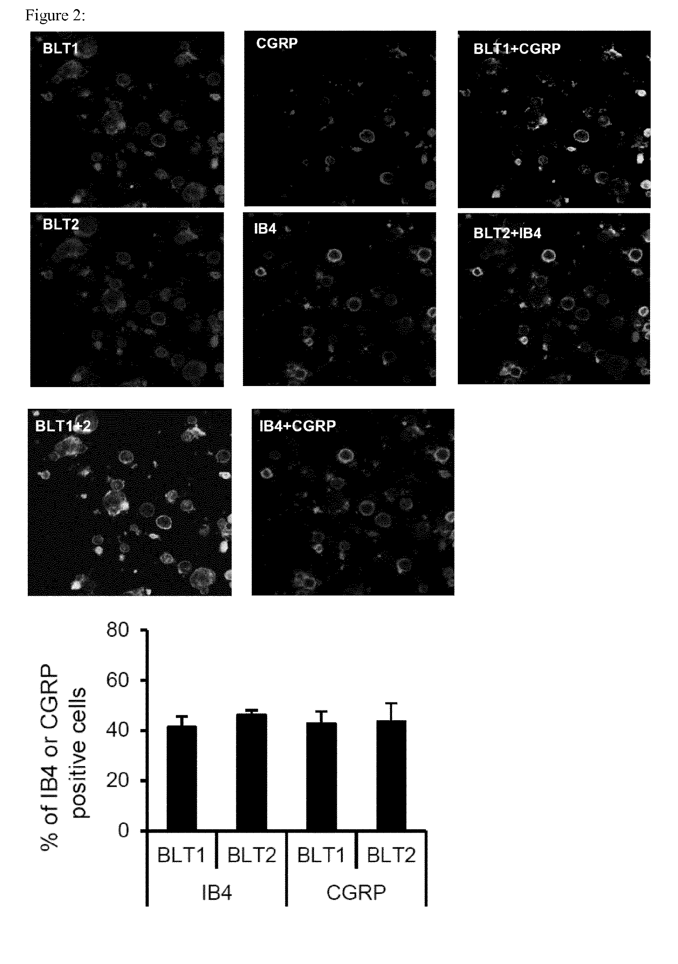

- **Figure 2:**: **BLT1 and 2 are co-expressed in peripheral sensory neurons.** Representative images of a MELC analysis from cultured DRG neurons 1 day after preparation. Images for BLT1, 2, the lectin IB4 and CGRP are shown in false colors. The white bar represents xx µm. Percentage of IB4- (n= 179) or CGRP- positive neurons (n=111) expressing BLT1 or 2.

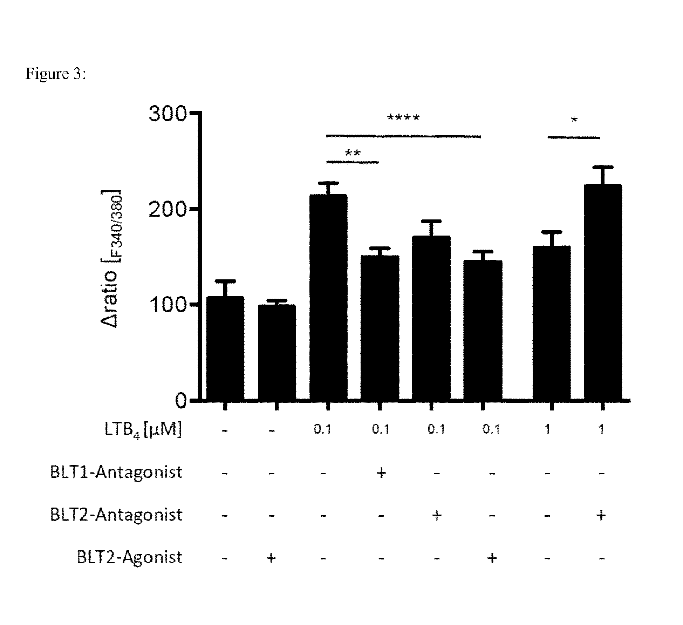

- **Figure 3:**: **BLT1 mediates TRPV1-sensitization and BLT2 mediates TRPV1-desensitization by LTB4.** (A) DRG neurons were double stimulated with capsaicin (200 nM, 15 s each) and incubated with either vehicle or the indicated compounds for two minutes prior to the second capsaicin stimulation. TRPV1 sensitization by LTB4 (100 nM or 1 µM) can be reduced by preincubation with a BLT1 antagonist (U73502, 1 µM) or by a BLT2 agonist (CAY10583, 400 nM) while a BLT2 antagonist (LY2552833, 10 µM) increases TRPV1 sensitization. Data represent mean ± SEM 20-105 cells. ANOVA Kruskal-Wallis test; Dunn's multiple comparisons test *p<0.05, **p<0.01, ****p<0.0001 student's t-test; n.s. not significant.

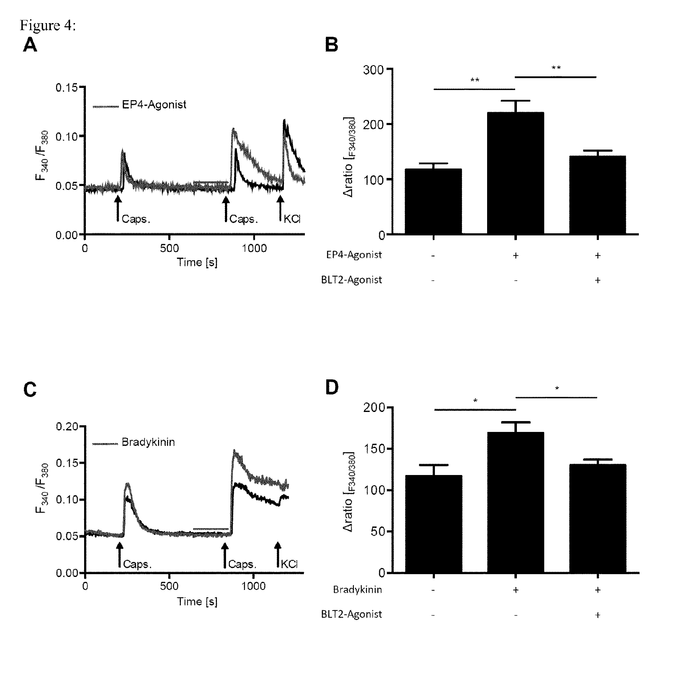

- **Figure 4:**: **BLT2 activation inhibits TRPV1-desensitization by EP4 and Bradykinin.** (A) DRG neurons were double stimulated with capsaicin (200 nM, 15 s each) and incubated with either the EP4 agonist ONOae329 (500 nM) or with ONOae329 and CAY10583 (400 nM) together for two minutes prior to the second capsaicin stimulation. (B) Difference in ratio between the first and the second capsaicin response using the same protocol as described in panel A. Data represent the mean ± SEM of the following number of neurons: 52 (vehicle), 109 (ONOae329), 106 (ONOae329 + CAY10583); One-Way ANOVA, Dunn's multiple comparisons test; ** p < 0,0096. (C+D) same as panel A+ B except that 500 nM bradykinin was used for TRPV1 sensitization. Data represent the mean ± SEM of the following number of neurons: 33 (vehicle), 83 (bradykinin), 93 (bradykinin + CAY10583); One-Way ANOVA, Holm-Sidak's multiple comparisons test; * p = 0,0126

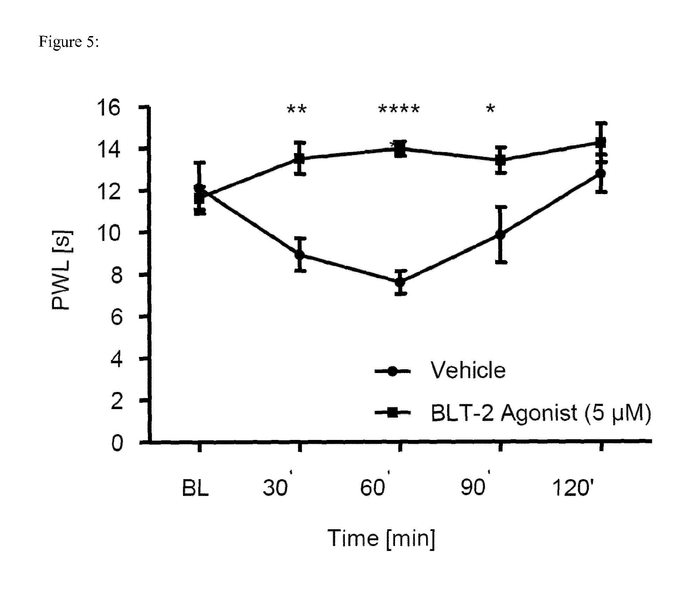

- **Figure 5:**: **BLT2 activation decreases thermal pain thresholds in mice.** CAY10583 (10 µl, 5 µM) or vehicle was administered intraplantarly. Thermal thresholds were determined at the indicated time points. Data are presented as mean ± S.E.M. of 6 animals. Two way ANOVA/Bonferroni *P<0.05, ** P<0.005, **** P<0.0005.

### EXAMPLES

### Materials and Methods

### Animals:

All animal experiments were performed according to the recommendations in the Guide for the Care and Use of Laboratory Animals of the National Institutes of Health and approved by the local Ethics Committees for Animal Research (Darmstadt). For all behavioral experiments the inventors used only 6-12 weeks old male C57BL/6N mice purchased from commercial breeding companies (Charles River, Sulzfeld, Germany, Janvier, Le Geneset-Saint-Isle, FR). To compare mechanical thresholds the inventors used age and sex matched littermates as control.

### Behavioral Tests:

For the determination of mechanical allodynia or thermal hypersensitivity, mice were kept in test cages on an elevated grid for at least 2 hours to allow accommodation. Baseline measurements were performed using a Hargreaves Apparatus (Ugo Basile, Comerio, VA, Italy) detecting withdrawal latency of the hind paws after mechanical stimulation for determination of thermal thresholds, mice were kept in test cages on a warmed glass plate (32°C) for at least 2 hours on the first day to allow accommodation. Then, the mid-plantar region of the paws was stimulated with a radiant heat device, consisting of a high intensity projector lamp, until withdrawal occurred. The non-injected and injected paws were measured alternately in intervals of 5-10 min. For all behavioral tests the investigator was blinded for treatment or genotype of the mice. 10 µl of LTB4 (5 µM), CAY10583 (5 µM) or vehicle were injected intraplantarly.

### Primary dorsal root ganglia (DRG) cultures:

Murine DRGs were dissected from spinal segments and directly transferred to ice cold HBSS with CaCl₂ and MgCl₂ (Invitrogen, Carsbad, CA, USA). Next, isolated DRGs were incubated with collagenase/dispase (500 U/ml Collagenase; 2.5 U/ml Dispase) in neurobasal medium containing L-glutamine [2 mM] penicillin (100 U/ml), streptomycin (100 µg/ml), B-27 and gentamicin (50 µg/ml) (all from Invitrogen, Carlsbad, CA, USA) at 37°C for 75 min. After removal of the collagenase/dispase-solution, cells were washed twice with neurobasal medium containing 10% FCS and incubated for 10 min with 0.05% trypsin (Invitrogen, Carlsbad, CA, USA). The washing steps were repeated and the cells were mechanically dissociated with a 1 ml Gilson pipette. Finally, the neurons were plated on poly-1-lysine (Sigma, Deisenhofen, Germany) coated glass cover slips and incubated with neurobasal medium containing L-glutamine [2 mM] penicillin (100 U/ml), streptomycin (100 µg/ml), B-27 and gentamicin (50 µg/ml) over night until assessment by calcium imaging.

### Calcium Imaging experiments:

Calcium-Imaging experiments were performed as published previously. Briefly, Leica Calcium-imaging setup was used, consisting of a Leica DMI 4000 b inverted microscope equipped with a DFC360 FX (CCD-) camera, Fura-2 filters and an N-Plan 10x/0.25 Ph1 objective (all from Leica Microsystems, Wetzlar, Germany). Images were taken every 2 seconds and processed with the LAS AF-software. For each experiment the inventors chose an area with large cell numbers and monitored 40 - 110 cells simultaneously. Calcium-Imaging experiments were performed using DRG-neurons 24 - 48 hours after preparation. Cells were loaded with 5 µM fura-2-AM-ester and 0.02% Pluronic F-127 (both Biotium, Hayward, CA and incubated for 30 to 60 min. at 37°C. Then, the cells were washed with external solution (containing in mM: NaCl [145], CaCl₂ [1.25], MgCl₂ [1], KCl [5], D-glucose [10], HEPES [10]; adjusted to pH 7.3). Baseline measurements were performed in external solution at a flow rate of 1 - 2 ml/min. Stimulation of the neurons were done using Capsaicin (200 nM). Where indicated a 2 minute pretreatment with LTB₄ (100-1000 nM), BLT2 Agonist CAY10583 (400 nM), BLT2 Antagonist LY2552833 (10 µM), or BLT1 Antagonist U75302 (1 µM) (all Cayman Chem. Ann Arbor, MI) was performed.

### Multi Epitope Ligand Cartography (MELC):

The MELC technology has been described previously (Pierre et al., 2008; Pierre, 2010). 10 µl LTB4, CAY10583 or vehicle were injected in the hindpaw. Animals were killed at the indicated time points. Paw edemas were embedded in tissue freezing medium (Leica microsystems Nussloch, Germany), cryosections of 10 µm thickness were sliced using the Leica CM 3050S cryostat (-20°C; Leica, Wetzlar, Germany) and applied on silane-coated coverslips. The tissue was fixed in 4% paraformaldehyde in PBS, permeabilized with 0.1% triton in PBS and blocked with 3% BSA in PBS for 1h at room temperature. The sample was placed on the stage of an inverted wide-field fluorescence microscope (Leica DM IRE2; x63 oil lens NA 1.32). A picture before the application of antibodies was taken. By a robotic process, first the slices were incubated for 15 min with predetermined fluorescence-labeled antibodies (supplementary data 1) and rinsed with PBS. Afterwards, the phase contrast and fluorescence signals were imaged by a cooled charge-coupled device camera (Apogee KX4; Apogee Instruments, Roseville, CA, 2x binning results in images of 1024x1024 pixels; final pixel size was 286x286 nm²). To delete the specific signal of the antibody before addition of the next, a bleaching step was performed. A postbleaching image was recorded and subtracted from the following fluorescence tag image during the data analysis. Using the corresponding phase contrast images, fluorescence images produced by each antibody were aligned pixel-wise. Images were corrected for illumination faults using flat-field correction.

### Data analysis and statistics:

All data are presented as mean ± S.E.M. To determine statistically significant differences in all behavioral experiments analysis of variance (ANOVA) for repeated measures was used followed by post hoc Bonferroni correction using GraphPad Prism. For *in vitro* experiments comparing only two groups, student's *t*-test was carried out. P < 0.05 was considered as statistically significant.

### Example 1: Increasing LTB4 Concentrations Reducing LTB4-mediated TRPV1 Sensitization

LTB4 has been shown to activate at high concentrations TRPV1. Here, the inventors investigated whether or not LTB4 is able to sensitize TRPV1 activation at lower concentrations. Therefore the inventors stimulated primary cultures of murine DRG neurons twice with the selective TRPV1-agonist capsaicin and incubated the cells prior the second stimulation with increasing LTB4 concentrations. The inventors found that preincubation with 100 nM LTB4 doubled the capsaicin-induced intracellular calcium increases (Fig. 1A,B). Surprisingly, the enhanced response to LTB4 was reduced when using higher LTB4 concentrations (200-1000 nM; Fig. 1B).

The narrow concentration range for LTB4-induced TRPV1 sensitization led us to hypothesize that both LTB4 receptors, the high affinity receptor BLT1 and the low affinity receptor BLT2, mediate opposing effects on TRPV1 sensitization. Thus, the sensitization seen at low LTB4 concentrations (100 nM) might be mediated by the high affinity LTB4 receptor BLT1. With increasing LTB4 concentrations the low affinity LTB4 receptor BLT2 might become activated and decrease the TPV1 sensitization.

### Example 2: Expression of BLT2 in Sensory Neurons

In the next step, the expression of BLT1 and BLT2 in DRG neurons was determined. Serial immunohistochemistry using the MELC system showed that BLT1 and 2 are colocalized in murine DRG neurons (Fig. 2). The BLT1 and 2 expressing neurons were sensory neurons, since they also expressed calcitonin gene related protein (CGRP), a marker for peptigergic sensory neurons, or isolectin B4 (IB4), a marker for non-peptigergic sensory neurons (Fig. 2). In total around 40% of all IB4 and CGRP-expressing neurons were also expressing BLT1 and 2.

### Example 3: BLT2 Agonist CAY10583 Abolishes LTB4-mediated TRPV1 Sensitization

To investigate whether or not BLT1 mediates TRPV1 sensitization, the inventors tested effect of the BLT1 antagonist U75302 on LTB4-induced TRPV1 sensitization. Indeed, U75302 was able to abolish TRPV1-sensitization induced by 100 nM LTB4 (Fig. 3). Next, the inventors studied the effect of the BLT2 antagonist LY2552833 on TRPV1 sensitization. In accordance with the notion that BLT2 decreases TRPV1 sensitization, LY2552833 had no significant effect on TRPV1 sensitization by 100 nM LTB4, but increased TRPV1 sensitization by 1000 nM LTB4 (Fig. 3). Finally, the BLT2 agonist CAY10583 by itself had no effect on capsaicin-induced TRPV1 activation, but abolished TRPV1 sensitization by 100 nM LTB4 (Fig. 3).

### Example 4: BLT2 Agonist CAY10583 Abolishes ONOae329- and PKC-mediated TRPV1 Sensitization

Next, the effect of the BLT2 agonist CAY10583 on TRPV1 sensitization by receptors activating signaling pathways known to sensitize TRPV 1 was studied. Therefore, TRPV 1 was sensitized using the PGE2 receptor 4 (EP4) ligand ONOae329. Here, sensitization is achieved through increased cAMP synthesis, subsequent protein kinase A (PKA) activation and TRPV1 phosphorylation (Bhave et al., 2002). Pretreatment of murine DRG neurons with ONOae329 induced a strong sensitization of TRPV1 responses to capsaicin which was abolished in presence of the BLT2 agonist CAY10583 (Fig. 4A,B). Likewise, bradykinin-induced phosphorylation and sensitization of TRPV1 through PKC was significantly decreased in presence of CAY10583 (Fig. 4C,D).

### Example 5: BLT2 Agonist CAY10583 Reduces Pain Sensation in Rats

To study potential analgesic effects of BLT2 activation, the BLT2 agonist CAY10583 or vehicle were injected in hind paws of mice and the thermal pain thresholds were determined. In accordance with the inventor's data showing decreased TRPV1 sensitization in vitro, CAY10583 was able to significantly increase thermal pain thresholds as compared to mice receiving only vehicle (Fig. 5).

### References

Andoh T, Kuraishi Y (2005) Expression of BLT1 leukotriene B4 receptor on the dorsal root ganglion neurons in mice. Brain research Molecular brain research 137:263-266.

Bhave G, Zhu W, Wang H, Brasier DJ, Oxford GS, Gereau RWt (2002) cAMP-dependent protein kinase regulates desensitization of the capsaicin receptor (VR1) by direct phosphorylation. Neuron 35:721-731.

Hwang SW, Cho H, Kwak J, Lee SY, Kang CJ, Jung J, Cho S, Min KH, Suh YG, Kim D, Oh U (2000) Direct activation of capsaicin receptors by products of lipoxygenases: endogenous capsaicin-like substances. Proc Natl Acad Sci U S A 97:6155-6160.

Iizuka Y, Yokomizo T, Terawaki K, Komine M, Tamaki K, Shimizu T (2005) Characterization of a mouse second leukotriene B4 receptor, mBLT2: BLT2-dependent ERK activation and cell migration of primary mouse keratinocytes. J Biol Chem 280:24816-24823.

Okubo M, Yamanaka H, Kobayashi K, Fukuoka T, Dai Y, Noguchi K (2010) Expression of leukotriene receptors in the rat dorsal root ganglion and the effects on pain behaviors. Mol Pain 6:57.

Pierre S, Maeurer C, Coste O, Becker W, Schmidtko A, Holland S, Wittpoth C, Geisslinger G, Scholich K (2008) Toponomics analysis of functional interactions of the ubiquitin ligase PAM (Protein Associated with Myc) during spinal nociceptive processing. Mol Cell Proteomics 7:2475-2485.

Pierre S, and Scholich, K. (2010) Toponomics: Studying protein-protein interactions and protein networks in intact tissue. Molecular BioSystems 6:641-647.

Scholich K, Geisslinger G (2006) Is mPGES-1 a promising target for pain therapy? Trends Pharmacol Sci 27:399-401.

Sisignano M, Park CK, Angioni C, Zhang DD, von Hehn C, Cobos EJ, Ghasemlou N, Xu ZZ, Kumaran V, Lu R, Grant A, Fischer MJ, Schmidtko A, Reeh P, Ji RR, Woolf CJ, Geisslinger G, Scholich K, Brenneis C (2012) 5,6-EET Is Released upon Neuronal Activity and Induces Mechanical Pain Hypersensitivity via TRPA1 on Central Afferent Terminals. J Neurosci 32:6364-6372.

Yokomizo T, Izumi T, Chang K, Takuwa Y, Shimizu T (1997) A G-protein-coupled receptor for leukotriene B4 that mediates chemotaxis. Nature 387:620-624.

Yokomizo T, Kato K, Terawaki K, Izumi T, Shimizu T (2000) A second leukotriene B(4) receptor, BLT2. A new therapeutic target in inflammation and immunological disorders. J Exp Med 192:421-432.

## Claims

1. A Leukotriene B4 receptor 2 (BLT2) agonist for use in the inhibition of a neurological sensation in a subject, preferably the prevention or treatment of pain, wherein the neurological sensation is mediated by the activation of transient receptor potential cation channel subfamily V member 1 (TRPV1).

2. The BLT2 agonist for use according to claim 1, which is CAY10583.

3. The BLT2 agonist for use according to claim 1 or 2, wherein said BLT2 agonist is used in combination with at least one additional compound inhibiting TRPV1-sensitization.

4. The BLT2 agonist for use according to any of claims 1 to 3, wherein said inhibition of a neurological sensation in a subject comprises the administration of the BLT2 agonist to the subject suspected to or experiencing the neurological sensation.

5. The BLT2 agonist according to any of claims 1 to 4, wherein said prevention or treatment of pain further comprises the administration of at least one additional compound inhibiting TRPV1-sensitization to the subject suspected to suffer from pain or suffering from pain.

6. The BLT2 agonist for use according to claim 3 or 5, wherein the at least one additional compound inhibiting TRPV1-sensitization is a Leukotriene B4 receptor 1 (BLT1) antagonist or inhibitor.

7. A combination for use in the prevention or treatment of pain, the combination comprising a BLT2 agonist and a BLT 1 antagonist or inhibitor.

8. The BLT2 agonist for use according to any of claims 1 to 6, or the combination for use according to claim 7, wherein said TRPV1 mediated sensation is a taste sensation, itching of the skin, a burning sensation, or a pain sensation.

9. The BLT2 agonist for use, or the combination for use according to claim 8, wherein the pain is inflammatory pain, inflammatory hyperalgesia, hyperalgesia, neuropathic pain, migraine, cancer pain, visceral pain, osteoarthritis pain, chronic pain and post-surgical pain.

10. A pharmaceutical composition for use in the prevention or treatment of pain, comprising a BLT2 agonist according to any of claims 1 to 6, or a combination according to claim 8.

11. The pharmaceutical composition for use according to claim 10, further comprising a pharmaceutically acceptable carrier and/or excipient.

12. An *in-vitro* method for desensitization of TRPV1 in a cell, the method comprising the step of activation of BLT2.

13. The method according to claim 12, wherein the activation of BLT2 in the cell is affected by contacting the cell with an effective amount of a BLT2 agonist.

14. The method according to claim 12 or 13, wherein the cell is a neuronal cell, preferably a peripheral neuronal cell.
